# EUROPEAN PATENT SPECIFICATION

(11) **EP 3 856 044 B1**
(45) Date of publication and mention of the grant of the patent: **13.08.2025**
(21) Application number: 19790951.8
(22) Date of filing: 30.09.2019
(51) Int. Cl.: A61B 17/04

(54) **SYSTEM FOR REPAIRING SOFT TISSUE TEARS**
SYSTEM ZUR REPARATUR VON WEICHTEILRISSEN
SYSTÈME POUR RÉPARER DES DÉCHIRURES DE TISSUS MOUS

(30) Priority: 28.09.2018 US 201862738551 P; 20.12.2018 US 201862782689 P; 01.03.2019 US 201962791127 P
(43) Date of publication of application: 04.08.2021
(60) Divisional application: 25180812.7
(73) Proprietor: CONMED Corporation, Largo, FL 33773 (US)
(72) Inventor: LOMBARDI, Giuseppe, New Port Richey, FL 34655 (US)
(74) Representative: Strehl & Partner mbB
(86) International application number: PCT/US2019/053727
(87) International publication number: WO 2020/069486

(56) References cited:
- EP-A1- 3 235 443
- EP-A1- 3 235 443
- US-A1- 2002 019 649
- US-A1- 2002 019 649
- US-A1- 2011 022 084
- US-A1- 2011 022 084
- US-A1- 2015 209 027
- US-A1- 2015 209 027
- US-A1- 2015 351 739
- US-A1- 2015 351 739

## Description

### BACKGROUND OF THE INVENTION

### 1. Field of the Invention

The present invention is directed generally to surgical tools and instruments and, more particularly, to a system for repairing soft tissue tears such as meniscal tears.

### 2. Description of Related Art

The meniscus is a piece of cartilage located within the knee joint, between the top of the tibia and the bottom of the femur. The meniscus serves to facilitate stable movement of the tibia and femur relative to one another, and to absorb shock and to spread load. The meniscus is frequently damaged (e.g., torn) as the result of injury and/or accident. A damaged meniscus can impede proper motion of the knee joint and cause pain, among other problems.

More particularly, the essential role of an intact meniscus, and its importance for proper knee function, has been well documented and accepted by the general orthopedic community. An intact and functioning meniscus is critical to optimally distribute weightbearing forces that transfer through the knee joint while maintaining knee stability. The meniscus is also vital to preserving the articular cartilage surfaces of the knee. Loss of meniscal tissue is considered to be a key precursor to the development of knee osteoarthritis.

A major challenge in repairing a torn meniscus is the fact that the tissue itself is a fibrous structure that is not uniformly vascular. The vascular zones of the meniscus comprise about one third of the meniscus tissue and are generally recognized as the "red-red" and "red-white" zones. The "red-red" zone (i.e., the most highly vascularized portion of the meniscus) is an area in which meniscal repairs are known to heal easily and is located along its outer periphery. The "red-white" zone extends from the most vascular area towards the inner portions of the meniscus where the blood supply eventually declines to nonvascular tissue (which is sometimes referred to as the "white-white" zone). It is believed that proper surgical technique is of great importance if a successful repair is to be achieved in the "red-white" zone. It is generally accepted knowledge that about 15% of all meniscal tears occur in the "red-red" zone, another 15% of meniscal tears occur in the "red-white" zone, and the remaining 70% of meniscal tears occur in the "white-white" (or non-vascularized) zone of the meniscus.

Another significant challenge in repairing a torn meniscus is that the size and shape of the tears vary, making the reduction and apposition of the torn tissue difficult to accomplish. Without proper apposition and stability, torn meniscal tissue will not heal properly.

The art of repairing torn meniscal tissue was first developed and pioneered throughout the 1980s by early sports medicine-focused surgeons. The earliest methods employed only suture in the repair. The techniques of "inside-out" and "outside-in" suturing became the so-called "gold standard for the repair of meniscal tissue. Both of these techniques focused on passing small diameter suture (size 2-0 or 3-0) through the meniscus, reducing and closing the tear, and then tying a suture knot over the knee capsule so as to fixate and stabilize the tear. A feature of these early all-suture repairs was that the surface of the meniscus was kept relatively smooth since the suture knot was outside of the knee joint, and the use of a needle and suture allowed the surgeon a great deal of flexibility in adequately reducing and stabilizing the tear.

Eventually, these early surgeons began concomitant use of complementary techniques to promote a vascular response in the more non-vascular areas of the meniscus. Methods such as tear edge and meniscapsular rasping, the application of an interpositional blood clot, trephination to create a vascular channel, and fascial sheath or synovial flap coverage have been shown in several studies to be 150% more effective in healing a torn meniscus when compared to repairs that do not use such concomitant techniques.

The specific issues and challenges associated with the aforementioned all-suture inside-out and outside-in repair techniques are centered primarily on issues relating to the "user interface" and to the "tethering" of the meniscus to the knee capsule. More particularly, the "user interface" issues generally relate to the technical demands required in the operating room: the skill of the surgeon and the number of assistants required to safely pass the needle and suture from the anterior portion of the meniscus through the posterior portion of the meniscus and exit out through the posterior/medial aspect of the knee joint (i.e., the so-called "inside-out" technique); or the passing of a needle and suture from the medial aspect of the exterior of the knee into the knee joint, through the meniscus, the retrieval and re-insertion back into the meniscus, and then passage back out through the capsule to the medial aspect of the knee (i.e., the so-called "outside-in" technique). The aforementioned tethering issues relate to more recent concerns about fixating suture over the knee capsule and thereby "tethering" the meniscus to the knee capsule, since evidence suggests that such tethering of the meniscus to the knee capsule may interfere with the normal biomechanics of the meniscus (e.g., load and force distribution, etc.).

As recognition of the importance of the meniscus grew in the late 1980s, new methods of meniscus repair were developed. These new methods focused on improving execution of the procedure in order to make it easier, simpler and faster to accomplish. The new gold standard approach became the so-called "all-inside" technique. The all-inside technique is intended to not violate the knee capsule or require any incisions on the posterior/medial aspects of the knee (i.e., such as is required with the inside-out and outside-in suturing techniques discussed above). With the all-inside technique, the entire repair both approximation and fixation is performed intraarticularly.

The first all-inside repair devices were tack-like implants that were inserted through a standard arthroscopic portal and then forcefully pushed through the meniscus, crossing through the tear, thereby closing and fixing the tear without the use of suture. These tack-like implants were formed out of biomaterials such as PLA, PLLA or PGA that were expected to biodegrade over time. However, these materials are quite hard when first inserted and, in use, were found to degrade or bioabsorb much more slowly than anticipated. Clinical use and follow-up have demonstrated the inherent risks associated with the use of tack-like implants within the knee joint, as numerous published studies have reported device failure which can lead to tear reformation, loose implants within the knee joint and articular cartilage damage. Furthermore, it can be challenging for the surgeon to adequately address various tear shapes and sizes using these tack-like implants.

As a result, attention has returned to suture-based repairs, with a new focus on performing a suture-based repair using an all-inside technique. There are several recent systems that seek to accomplish this goal. An anchor system from which the pre-characterising first part of claim 1 starts out is disclosed in EP 3235443 A1. Related art is disclosed in US 2011/022084 A1, US 2015/351739 A1 and US 2002/019649 A1. However, none of these systems have been found to be completely satisfactory.

Thus, there is a need for a new and improved apparatus for meniscal repair.

### SUMMARY OF THE INVENTION

The above object is solved by an anchor system as set forth in the appended claims. Embodiments of the present invention are directed to a system for repairing soft tissue tears such as meniscal tears. According to one aspect, the anchor system has a first implant connected to a length of suture. The length of suture is folded such that a tensioning limb extends from the first implant and a locking limb extends from the first implant. The anchor system also includes a second implant fixed to the locking limb and an adjustment mechanism in the length of a suture between the first implant and the second implant. The tensioning limb is passed through the adjustment mechanism.

Also disclosed herein is a delivery device. The delivery device includes an elongated body with a needle extending distally therefrom and a pusher assembly within the elongated body. The pusher assembly has a cannulated pusher rod extending distally from a pusher body and an actuator extending from the pusher body through the elongated body. The cannulated pusher rod is slidable within the needle. The delivery device also includes a locking mechanism on the elongated body which is movable between a locked position and unlocked position. The actuator is moveable from a first configuration to a second configuration when the locking mechanism is in the locked position and the actuator is moveable from the second configuration to a third configuration when the locking mechanism is in the unlocked position.

Also disclosed herein is a method for meniscal repair, which is not part of the invention. The method includes the steps of: (i) providing a delivery device an elongated body with a needle extending distally therefrom, a pusher assembly within the elongated body, the pusher assembly comprising a cannulated pusher rod extending distally from a pusher body and an actuator extending from the pusher body through the elongated body, wherein the cannulated pusher rod is slidable within the needle, and a locking mechanism on the elongated body, the locking mechanism movable between a locked position and unlocked position; (ii) providing an anchor system having a first implant connected to a length of suture, the length of suture folded such that a tensioning limb extends from the first implant and a locking limb extends from the first implant, a second implant fixed to the locking limb, and an adjustment mechanism in the length of suture between the first implant and the second implant, wherein the tensioning limb is passed through the adjustment mechanism; (iii) positioning the needle at a first piercing location on a first side of a tissue; (iv) piercing the needle through the first piercing location to a second side of the tissue; (v) moving the actuator distally, deploying the first implant from the delivery device; (vi) removing the needle from the first piercing location on the first side of the tissue; (vii) positioning the needle at a second piercing location on a second side of the tissue; (viii) piercing the needle through the first piercing location to a second side of the tissue; (ix) moving the locking mechanism from the locked position to the unlocked position; and (x) moving the actuator farther distally, deploying the second implant from the delivery device.

These and other aspects of the invention will be apparent from and elucidated with reference to the embodiment(s) described hereinafter.

### BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:
FIG. 1 is a side perspective view schematic representation of an anchor system, according to an embodiment;
FIG. 2 is a perspective view schematic representation of the implant of the anchor system, according to an embodiment;
FIG. 3A is a perspective view schematic representation of the first step in creating a pierce hitch in the suture, according to an embodiment;
FIG. 3B is a perspective view schematic representation of the second step in creating a pierce hitch in the suture, according to an embodiment;
FIG. 3C is a perspective view schematic representation of the third step in creating a pierce hitch in the suture, according to an embodiment;
FIG. 3D is a perspective view schematic representation of the final step in creating a pierce hitch in the suture, according to an embodiment;
FIG. 4 is a perspective view schematic representation of the length of suture connected to the second implant, according to an embodiment;
FIG. 5 is a perspective view schematic representation of the anchor system in a pre-deployment configuration, according to an embodiment;
FIG. 6 is a perspective view schematic representation of the anchor system in the pre-deployment configuration, according to an embodiment;
FIG. 7 is a side view schematic representation of an anchor system, according to an alternative embodiment;
FIG. 8 is a perspective view schematic representation of the anchor system of FIG. 7;
FIG. 9 is another perspective view schematic representation of the anchor system of FIG. 7;
FIG. 10 is a perspective view schematic representation of an anchor system, according to another alternative embodiment;
FIG. 11 is a perspective view schematic representation of a delivery device, according to an example;
FIG. 12 is a perspective view schematic representation of a pusher assembly of the delivery device, according to an example;
FIG. 13 is a side view schematic representation of the delivery device in a first configuration, according to an example;
FIG. 14 is a side view schematic representation of the delivery device in a second configuration, according to an example;
FIG. 15 is a side view schematic representation of the delivery device with the locking mechanism in the unlocked position, according to an example;
FIG. 16 is a side view schematic representation of the delivery device in a third configuration, according to an example;
FIG. 17 is a side view schematic representation of the delivery device in a fourth configuration, according to an example;
FIG. 18 is a top view schematic representation of a delivery device, according to an alternative example;
FIG. 19 is a side view schematic representation of the delivery device, according to an alternative example;
FIG. 20 is a sectional side view schematic representation of the delivery device, according to an alternative example;
FIG. 21 is a sectional side view schematic representation of the delivery device in a first configuration, according to an alternative example;
FIG. 22 is a sectional side view schematic representation of the delivery device in a second configuration, according to an alternative example;
FIG. 23 is a top view schematic representation of the anchor system in a deployed configuration, according to an embodiment;
FIG. 24 is a top perspective view schematic representation of the anchor system in a deployed configuration, according to another embodiment;
FIG. 25 is a top perspective view schematic representation of the anchor system in a deployed configuration, according to an alternative embodiment;
FIG. 26 is a side perspective view schematic representation of the anchor system in the deployed configuration, according to an embodiment;
FIG. 27 is a top perspective view schematic representation of the anchor system in the deployed configuration, according to an embodiment;
FIG. 28 is a top perspective view schematic representation of the anchor system in the deployed configuration, according to an embodiment;
FIG. 29 is a top perspective view schematic representation of the delivery device deploying the anchor system, according to an embodiment;
FIG. 30 is a top perspective view schematic representation of the delivery device deploying the anchor system, according to an embodiment;
FIG. 31 is a top perspective view schematic representation of the delivery device deploying the anchor system, according to an embodiment;
FIG. 32 is a top perspective view schematic representation of the delivery device deploying the anchor system, according to an embodiment;
FIG. 33 is a top perspective view schematic representation of the delivery device deploying the anchor system, according to an embodiment;
FIG. 34 is a top perspective view schematic representation of the delivery device deploying the anchor system, according to an embodiment;
FIG. 35 is a top perspective view schematic representation of the delivery device deploying the anchor system, according to an embodiment;
FIG. 36 is a top perspective view schematic representation of the anchor system in the deployed configuration, according to an embodiment;
FIG. 37 is a top perspective view schematic representation of the anchor system in the deployed configuration, according to an embodiment; and
FIG. 38 is a top perspective view schematic representation of the anchor system in the deployed configuration, according to an embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the underlying inventive concepts will be apparent to those skilled in the art from this disclosure.

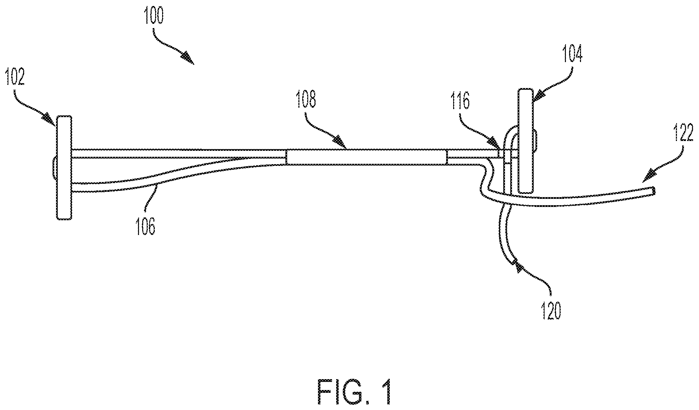

Referring now to the figures, wherein like reference numerals refer to like parts throughout, FIG. 1 shows a side perspective view schematic representation of an anchor system 100, according to an embodiment. The anchor system 100 includes a first implant 102 and a second implant 104 interconnected by a length of suture 106. The length of suture 106 terminates in a first end 120 and a second end 122. The length of suture 106 is used to adjust the position of the first implant 102 relative to the second implant 104 (and/or vice versa) by creating an one-way adjustable loop via an adjustment mechanism 108 and a locking mechanism 116. In some embodiments, the adjustment mechanism 108 is an eye splice (i.e., finger trap), while in other embodiments, the adjustment mechanism 108 is a sliding knot. The adjustment mechanism 108 can be any one-way adjustable locking construct. In an embodiment, the locking mechanism 116 is a pierce hitch. However, the locking mechanism 116 can be any fixed locking construct, such as a knot.

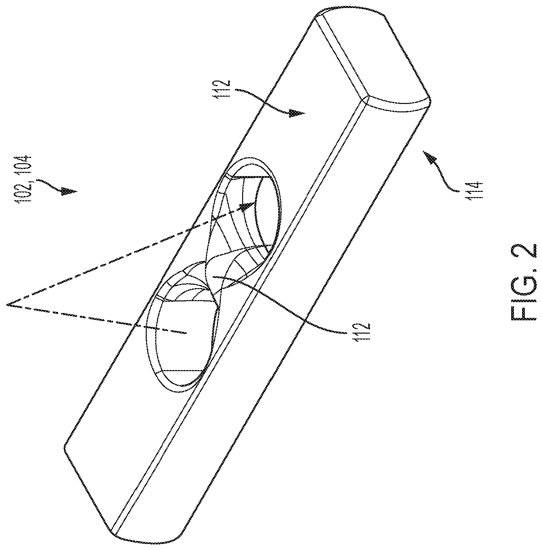

As shown in FIG. 2, the first and second implants 102, 104 are rectangular so they lay flat against the damaged tissue. However, the implants 102, 104 may have any other geometric configuration. The first and second implants 102, 104 can be composed of suture material, plastic, or any other suitable surgical material. As shown in FIG. 2, each implant 102, 104 has a pair of adjacent, spaced apertures 110. In the depicted embodiment, the pair of adjacent, spaced apertures 110 extend through a first side 112 of the implant 102, 104 to a second side 114 of the implant 102, 104. The implant 102, 104 has a radiused saddle 112 between the apertures 110 to maintain the suture 106 thereacross when the anchor system 100 is in a deployed configuration.

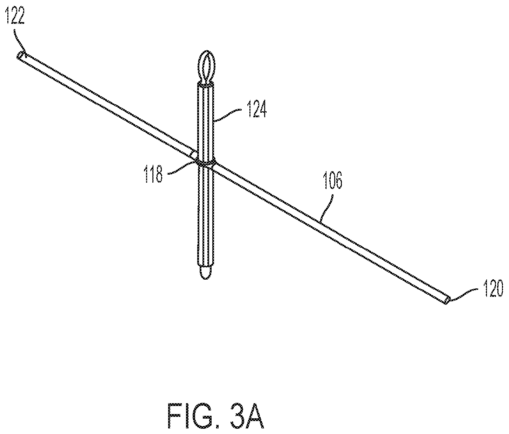

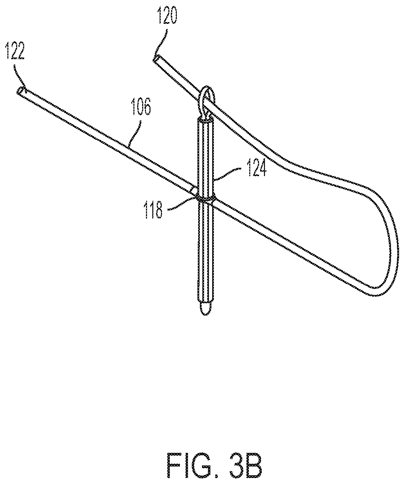

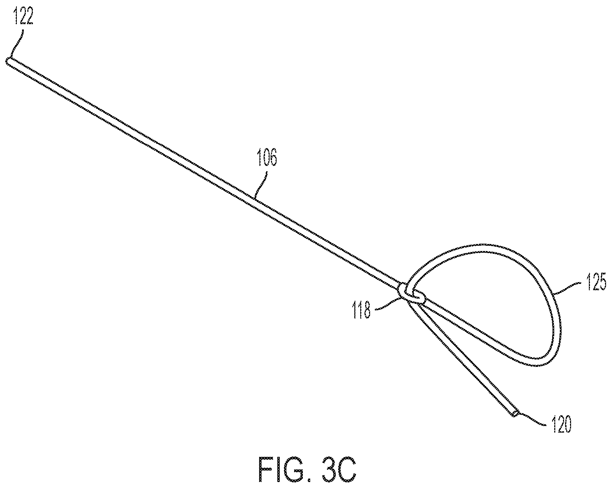

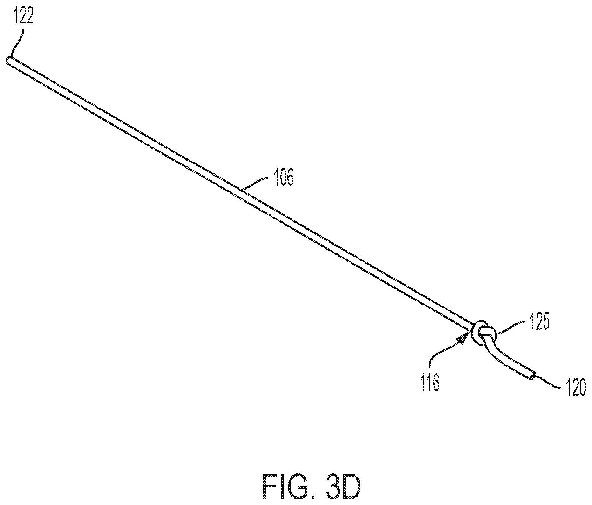

Turning now to FIGs. 3A-3D, there are shown perspective views schematic representations of a method for creating the locking mechanism 116 in the length of suture 106, according to an embodiment. In the embodiment shown in FIGs. 3A-3D, the locking mechanism 116 is a pierce hitch. To create a pierce hitch 116, a hole 118 is formed in the length of suture 106 between the first end 120 and the second end 122. A threader 124 or another similar device is placed through the hole 118 in the length of suture 106, as shown in FIG. 3A. Thereafter, the first end 120 of the suture 106 is threaded or woven through the threader 124, as shown in FIG. 3B. Then, the threader 124 is pulled through the hole 118 in the length of suture 106, creating a loop 125 in the suture 106, as can be seen in FIG. 3C. To minimize the loop 125, thereby creating the pierce hitch 116 shown in FIG. 3D, the first end 120 of the suture 106 is tensioned.

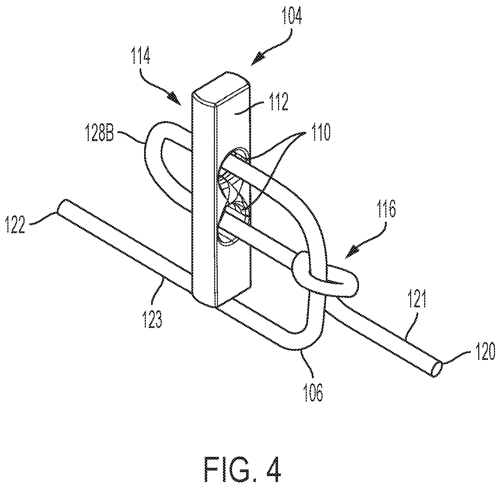

Referring now to FIG. 4, there is shown a perspective view schematic representation of the length of suture 106 connected to the second implant 104, according to an embodiment. In the depicted embodiment, the second implant 104 comprises the pair of adjacent, spaced apertures 110. The second end 122 of the length of suture 106 is first passed through one of the adjacent, spaced apertures 110 from a first side 112 of the implant 104. Then, the second end 122 of the length of suture 106 is passed through the other of the adjacent, spaced apertures 110 from a second side 114 of the implant 104 such that an intermediate portion 128B of suture 106 extends between the adjacent, spaced apertures 110 on the second side 114 of the implant 104 (over the radiused saddle 112). Finally, the second end 122 of the length of suture 106 is passed through the pierce hitch 116, as shown in FIG. 4, resulting in a "pierce hitch tail" 121 extending to the first end 120 of the suture 106 and a "tensioning limb" 123 extending to the second end 122 of the suture 106. The pierce hitch 116 functions as a self-collapsing noose around the pierce hitch tail 121 and is stationary relative to the position of the adjustment mechanism 108 (FIG. 1).

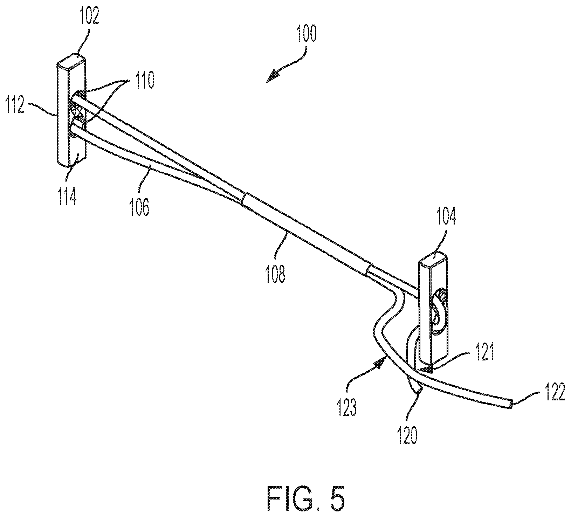

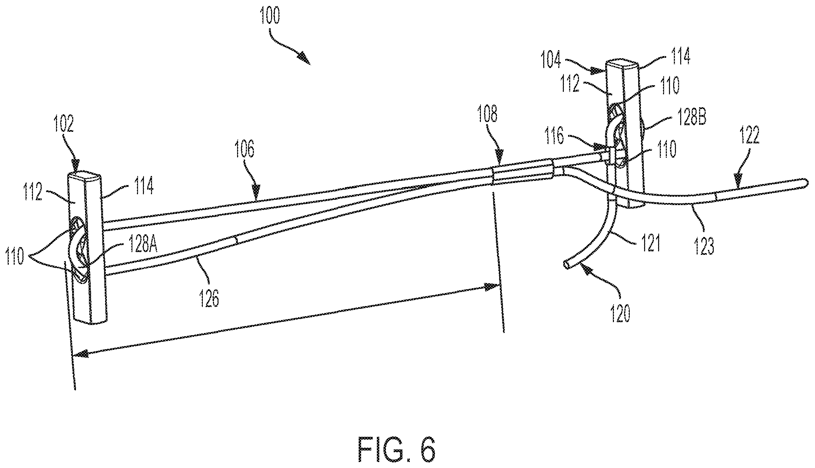

Turning now to FIG. 5, there is shown a perspective view schematic representation of the anchor system 100, according to an embodiment. From the configuration shown in FIG. 4, the first implant 102 is connected to the length of suture 106. The second end 122 of the length of suture 106 is passed through one of the pair of adjacent, spaced apertures 110 from the second side 114 of the implant 102. Then, the second end 122 of the length of suture 106 (i.e., tensioning limb 123) is passed through the other of the adjacent, spaced apertures 110 from the first side 112 of the implant 102 such that an intermediate portion 128A (FIG. 6) of the suture 106 extends between the adjacent, spaced apertures 110 on the first side 112 of the implant 102 (over the radiused saddle 112). Finally, the second end 122 of the length of suture 106 (i.e., tensioning limb 123) is passed through the adjustment mechanism 108. The tensioning limb 123 is slidable relative to the pierce hitch tail 121 (as the pierce hitch tail 121 does not slide).

Referring now to FIG. 6, there is shown a perspective view schematic representation of the anchor system 100, according to an embodiment. As shown in the depicted embodiment, the adjustment mechanism 108 is an eye splice. The eye splice 108 is formed in the length of suture 106 and the second end 122 of the length of suture 106 (i.e., tensioning limb 123) is passed through the eye splice 108, creating an adjustment loop 126 in the suture 106 between the first implant 102 and the eye splice 108. Thus, in the pre-deployment configuration, the intermediate portion 128A extends between the apertures 110 on the first side 112 of the first implant 102 and the intermediate portion 128B extends between the apertures 110 on the second side 114 of the second implant 104 with the eye splice 108 and the pierce hitch 116 therebetween. To adjust the adjustment loop 126 (i.e., change its diameter), the second end 122 of the suture 106 is pulled, moving the first implant 102 and second implant 104 closer together.

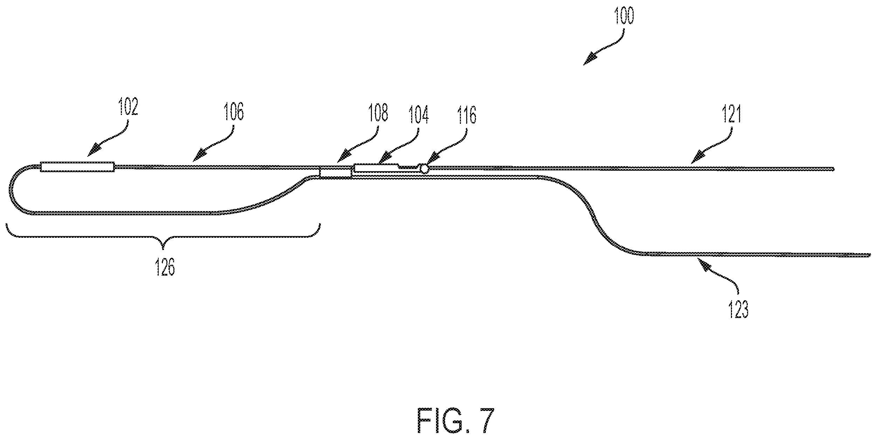

Turning now to FIGs. 7-10, there are shown various views schematic representations of the anchor system 100, according to alternative embodiments. FIG. 7 shows a side view of the anchor system 100 according to an alternative embodiment wherein the first and second implants 102, 104 are cannulated anchors (or any other tubular constructs). The cannulated anchors 102, 104 are composed of soft material, such as suture material. In the depicted embodiment, a length of suture 106 is folded in half, creating a sliding limb 123 and a non-sliding limb 121 (i.e., a tensioning limb 123 and a pierce hitch tail 121).

Still referring to FIG. 7, the length of suture 106 is passed through the first and second cannulated anchors 102, 104. A knot 116 is used to affix the second cannulated anchor 104 to the non-sliding limb 121. An eye splice 108 is formed in the non-sliding limb 121 between the first and second cannulated anchors 102, 104. The first cannulated anchor 102 is slidably connected to the suture 106 (the sliding limb 123). The sliding limb 123 is then passed through the eye splice 108, creating an adjustment loop 126, as shown in FIG. 7.

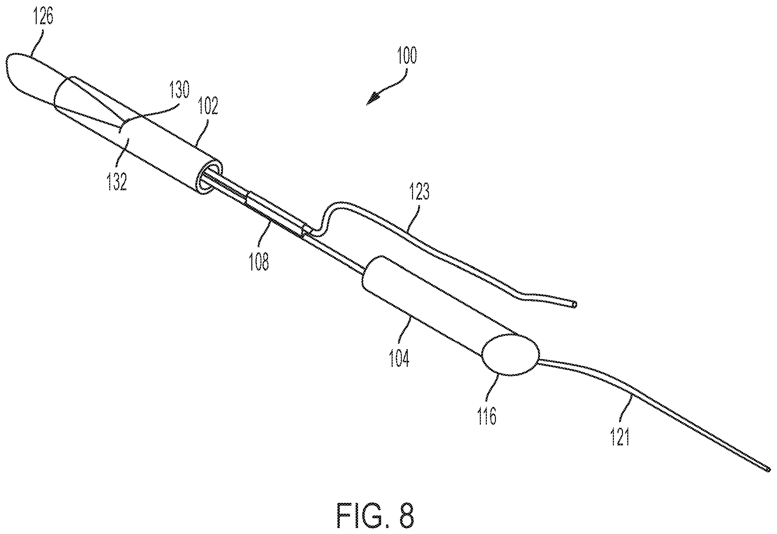

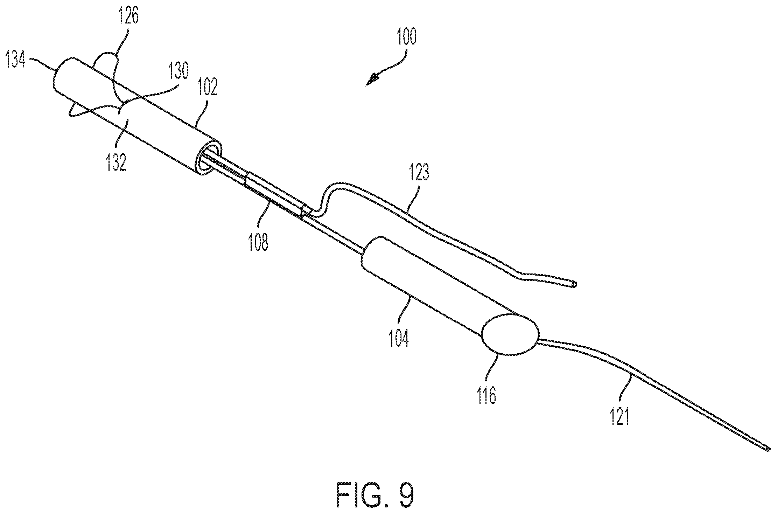

FIGs. 8 and 9 show perspective views schematic representations of the anchor system 100 of FIG. 7. In FIG. 8, the first cannulated anchor 102 is slidably attached to the suture 106 by passing the adjustment loop 126 through a slit 130 (or another type of segment) in the first cannulated anchor 102. Specifically, as shown in FIG. 8, the adjustable loop segment 102 is passed through the slit 130 in the first cannulated anchor 102 and exited out of a side 132 of the anchor 102. Then, the adjustable loop segment 126 is passed over a distal end 134 of the first cannulated anchor 102, thereby lassoing it, as shown in FIG. 9.

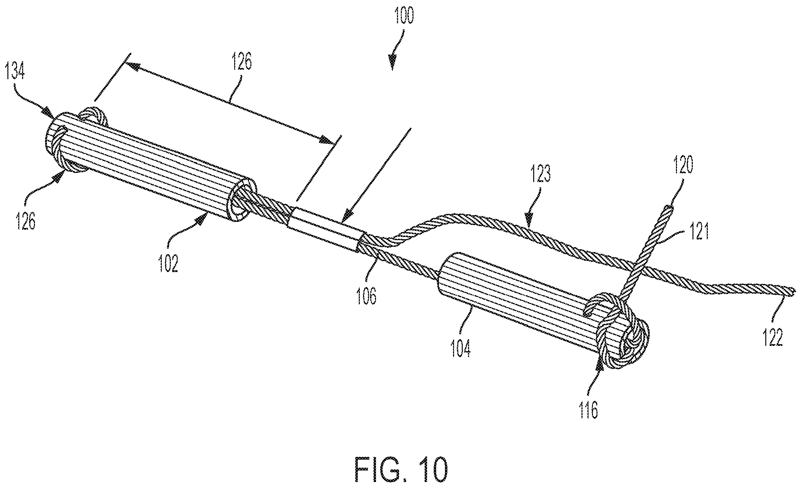

FIG. 10 shows a perspective view schematic representation of an anchor system 100, according to another alternative embodiment. The implants 102, 104 in FIG. 10 are also cannulated anchors (or any other tubular constructs). In the depicted embodiment, the cannulated anchors 102, 104 are composed of braided material. A length of suture 106 adjustably connects the first cannulated anchor 102 and the second cannulated anchor 104. In an embodiment, the length of suture 106 is a 2-0 suture, although other types of suture can be used. The length of suture 106 is folded in half so that both ends 120, 122 terminate on the same side and the other end forms a "U" shape (as also shown in FIG. 7). The second cannulated anchor 104 is secured to the suture 106 via a knot 116 in the length of suture 106. As with the previous embodiments of the anchor system 100, an eye splice 108 is formed in a locking limb 121 (also referred to herein as a non-sliding limb or pierce hitch tail) of the length of suture 106. The other limb, a tensioning limb 123, is passed through the eye splice 108, forming an adjustment loop 126. Similar to the anchor system 100 shown in FIGs. 8 and 9, the adjustment loop 126 extends around the distal end 134 of the first cannulated anchor 102.

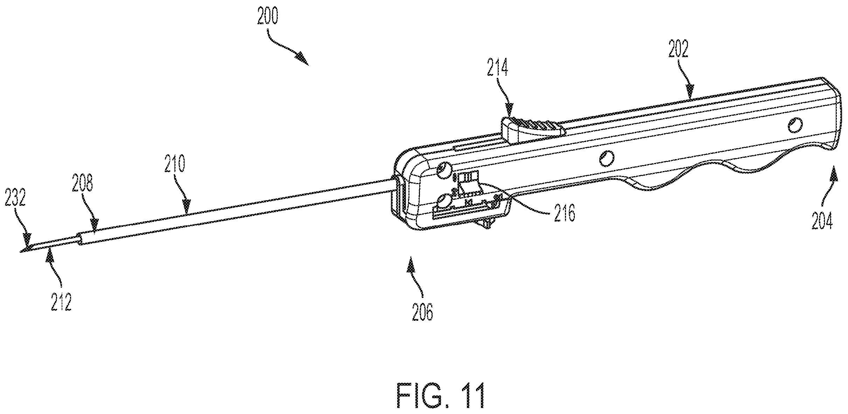

Referring now to FIGs. 11-17, there are shown various views schematic representations of a delivery device 200, according to an example. The delivery device 200 is configured to store one or more anchor systems 100 therein for deployment. FIG. 11 shows a perspective view of the delivery device 200. The delivery device 200 comprises an elongated body 202 having a proximal end 204 and a distal end 206. The delivery device 200 comprises an adjustable introducer sleeve 210 and a stop (not shown) with a positive locking mechanism (not shown) extending from the distal end 206 of the elongated body 102. A needle 212 extends distally from within the introducer sleeve 210. The anchor system 100 (FIG. 6) is disposed completely within the needle 212 of the delivery device 200. A cannulated pusher rod 208 extends within a lumen 232 of the needle 212 of the delivery device 200. The purpose of the cannulated pusher rod 208 is to push the anchor system 100 from the delivery device 200, as described in detail below. The elongated body 202 additionally comprises an actuator 214 and a locking mechanism 216.

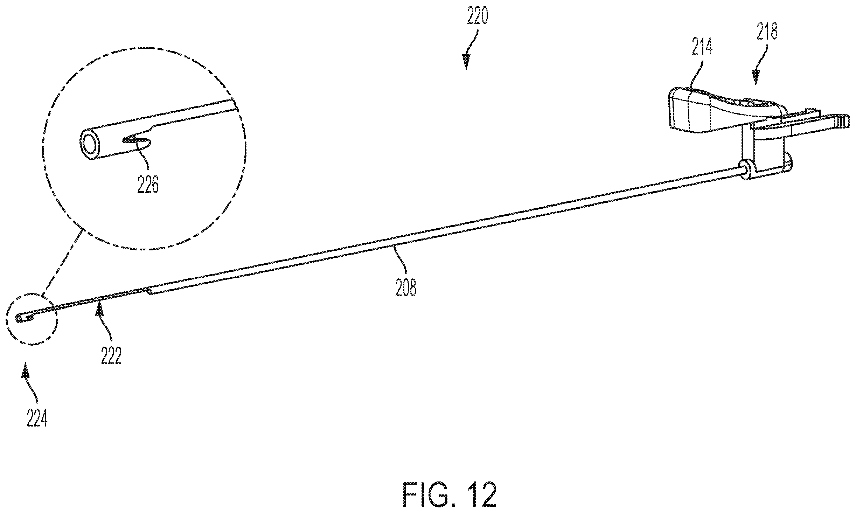

Turning now to FIG. 12, there is shown a perspective view schematic representation of a pusher assembly 220 of the delivery device 200, according to an example. The pusher assembly 220 comprises a pusher body 218 with the cannulated pusher rod 208 extending distally therefrom. The pusher body 218 comprises the actuator 214. In the depicted example, the actuator 214 is a thumb slide. The cannulated pusher rod 208 comprises a relieved area 222 between a distal end 224 of the pusher assembly 220 and the pusher body 218. The relieved area 222 is where a portion of the cannulated pusher rod 208 has been removed. The relieved area 222 allows for ease of rounding a corner or bend formed in the needle 212.

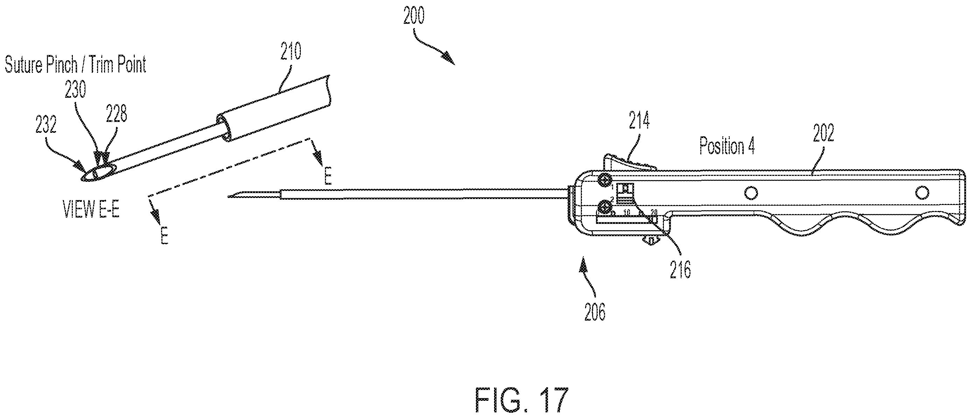

Still referring to FIG. 12, the cannulated pusher rod 208 also comprises a slit 226 at the distal end 224 of the pusher assembly 220. As shown, the slit 226 extends into the distal end 224 of the cannulated pusher rod 208 from the relieved area 222. This allows an end 120, 122 of suture 106 to be positioned within the slit 226. The cannulated pusher rod 208 can then be drawn proximally toward an opening 228 (FIG. 17) of the needle 212, creating a pinch point 230 between the cannulated pusher rod 208 and the lumen 232 of the needle 212 and severing an end 120, 122 of the suture 106.

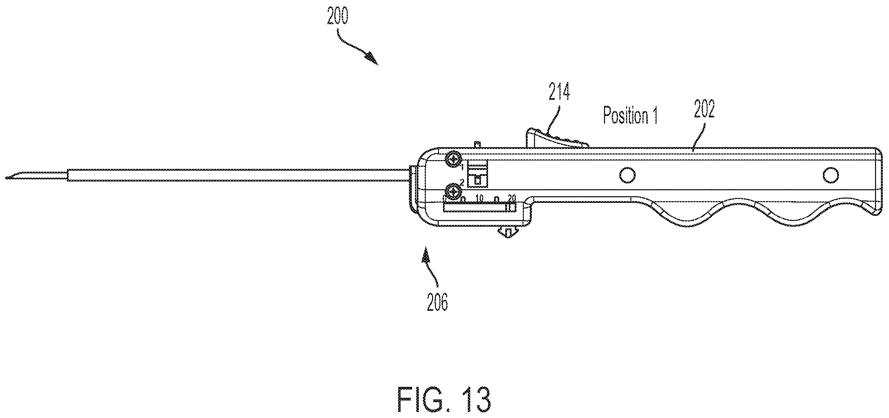

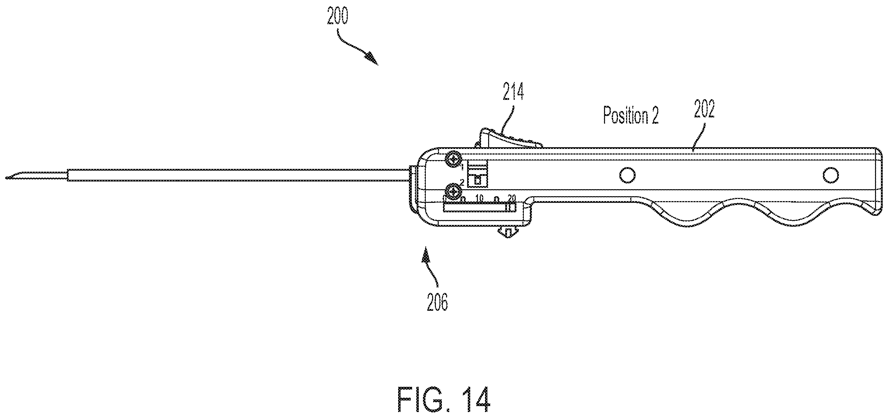

Turning now to FIGs. 13-17, there are shown side views schematic representations of the delivery device 200 in numerous configurations, according to an example. In the first configuration, the actuator 214 is a first distance from the distal end 206 of the elongated body 202, as shown in FIG. 13. In the first configuration, delivery device 200 is prepared to deploy the anchor system 100. In the second configuration, the actuator 214 is a second distance from the distal end 206 of the elongated body 202, as shown in FIG. 14. In an example, the first distance is greater than the second distance. When moving from the first configuration to the second configuration, the first implant 102 is deployed. Thus, by moving the actuator 214 (e.g., thumb slide) distally along the elongated body 202, the first implant 102 is deployed.

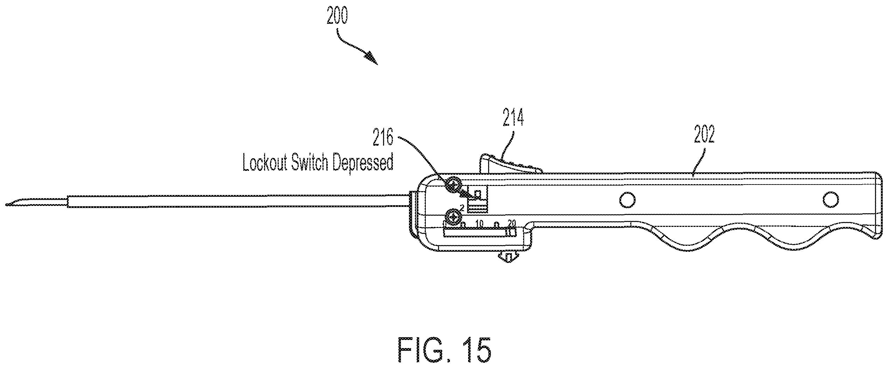

In both the first and second configurations, in FIGs. 13 and 14, the locking mechanism 216 is in the locked position. In the depicted example, the locking mechanism 216 is a lockout switch. The lockout switch 216 is slidable or otherwise movable between a locked position and an unlocked position. In the locked position, as shown in FIGs. 13 and 14, the actuator 214 cannot advance distally from the second configuration in FIG. 14. The actuator 214 cannot advance distally along the elongated body 202 past the lockout switch 216. The lockout switch 216 can be depressed and moved to the unlocked position, as shown in FIG. 15

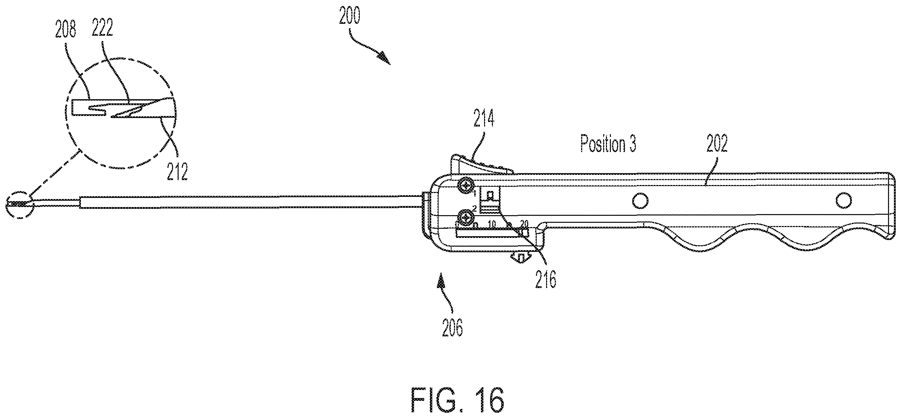

When the lockout switch 216 is in the unlocked position, as shown in FIG. 15, the actuator 214 can move distally to a third distance from the distal end 206 of the elongated body 202 to achieve the third configuration shown in FIG. 16. When moving from the second configuration to the third configuration, the pusher rod 208 is extended out of the needle 212, deploying the second implant 104. In the third configuration, the relieved area 222 is exposed between the pusher rod 208 and the needle 212, as shown.

In the third configuration, suture 106 can be moved into the relieved area 222 between the pusher rod 208 and the needle 212. With the end(s) 120, 122 of suture 106 extending through the relieved area 222, the delivery device 200 can be moved from the third configuration to a fourth configuration. To move the delivery device 200 from the third configuration to the fourth configuration, the actuator 214 is pulled in the proximal direction. When the delivery device 200 moves from the third configuration to the fourth configuration (FIG. 17), the cannulated pusher rod 208 is drawn back into the needle 212, allowing the end(s) 120, 122 of suture 106 to be cut against needle 212.

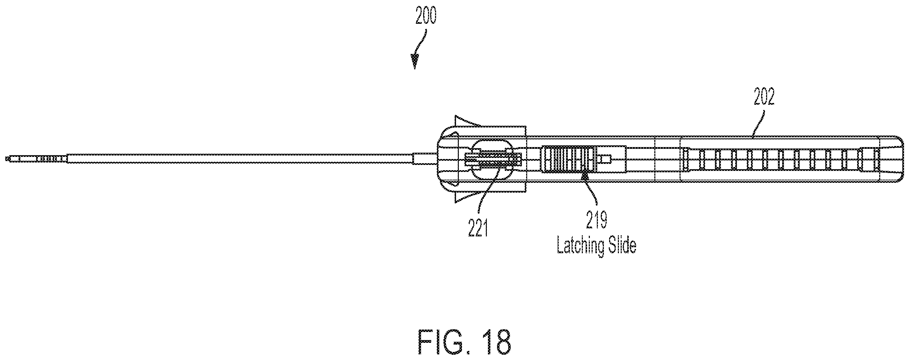

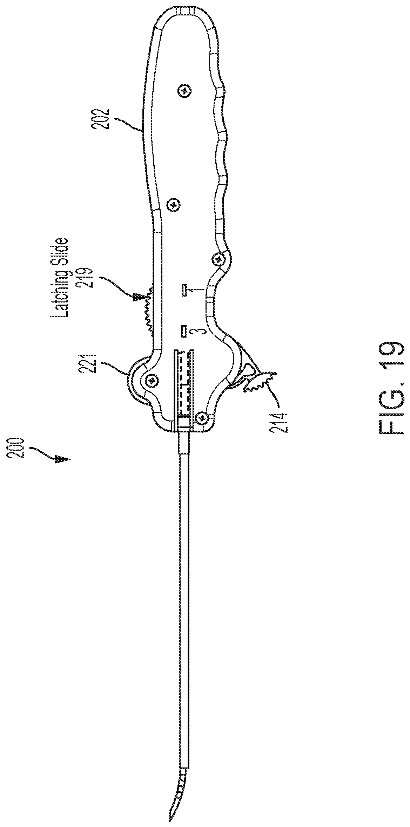

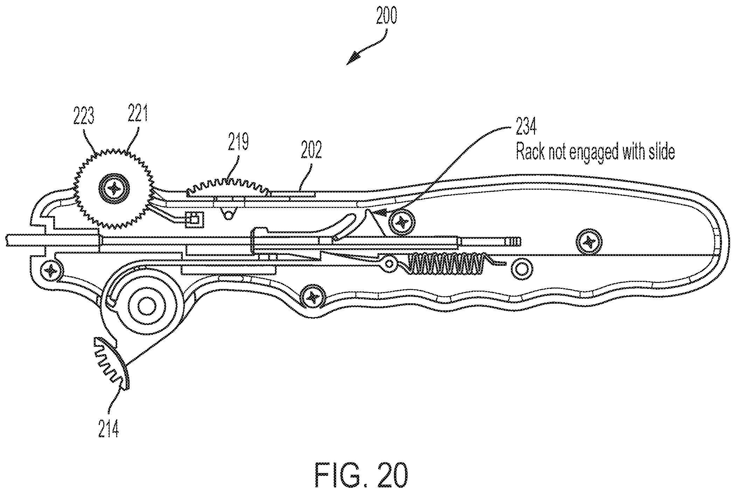

Referring now to FIGs. 18-22, there are shown various views schematic representations of a delivery device 200, according to an alternative example. FIGs. 18 and 19 show an optional latching slide 219 and a tensioning wheel 221 of the elongated body 202 of the delivery device 200. The delivery device 200 also includes the actuator 214 on an opposing side of the elongated body 202 relative to the latching slide 219. As shown in FIG. 20, a rack 234 within the elongated body 202 connects to the cannulated pusher rod 208 and moves the cannulated pusher rod 208 in and out from the needle 212.

Still referring to FIG. 20, the actuator 214 comprises a ratchet mechanism for selectively advancing the cannulated pusher rod 208. When the actuator 214 is pulled in the proximal direction, the rack 234 is moved distally, driving the cannulated pusher rod 208 in the distal direction. The actuator 214 may have a spring return such that the actuator 214 returns back to its first (or starting) configuration when released. The tensioning wheel 221 extends partially through the elongated body 202 such that suture 106 extending within the needle 212 can wrap around the tensioning wheel 221. The tensioning wheel 221 provides traction on the suture 106 when the implants 102, 104 are deployed. In the depicted example, the tensioning wheel 221 comprises teeth 223 for gripping the suture 106. The latching slide 219 is optional and can be used to selectively lock the tensioning wheel 221 against rotation.

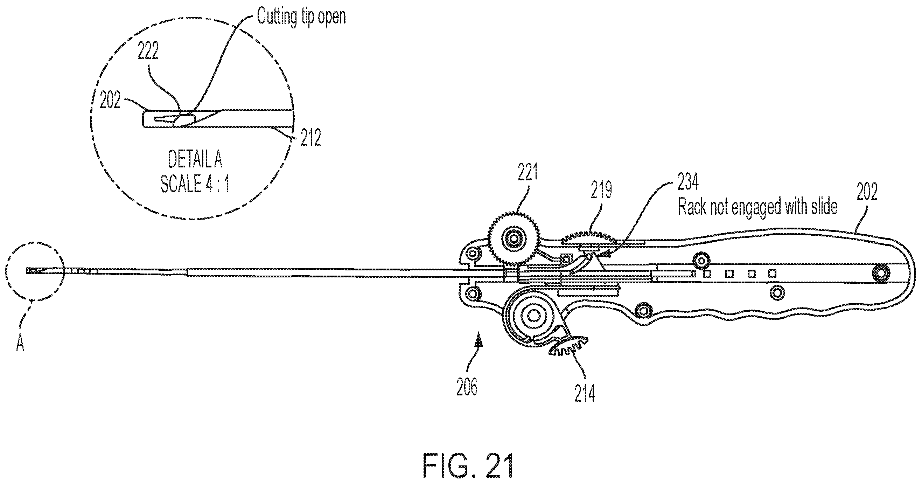

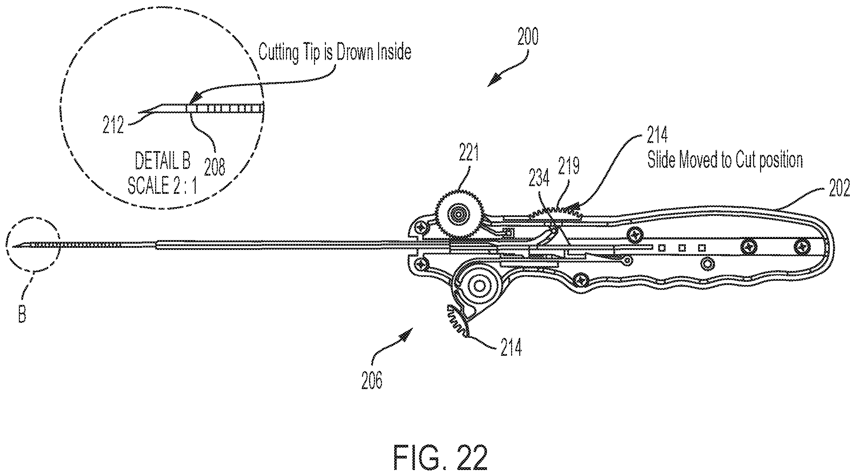

In a first configuration, as shown in FIGs. 19 and 22, the cannulated pusher rod 208 extends within the needle 212. The delivery device 200 can then be moved from the first configuration to a second configuration, shown in FIG. 21. To move the delivery device 200 from the first configuration to the second configuration, the actuator 214 is squeezed or pulled in the proximal direction. Moving the actuator 214 proximally, drives the rack 234 distally and as a result, pushes the cannulated pusher rod 208 distally out from the needle 212, expelling the first implant 102 from the needle 212. The actuator 214 can be released and then pulled proximally again when it is time to expel and deploy the second implant 104. The actuator 214 can also be maintained in the second configuration (FIG. 21) by holding the actuator 214 proximally, exposing the relieved area 222. With the relieved area 222 exposed, the ends 120, 122 of suture 106 can be extended into the relived area 222. When the actuator 214 is then released (FIG. 22), the end(s) 120, 122 of suture 106 are cut against the needle 212.

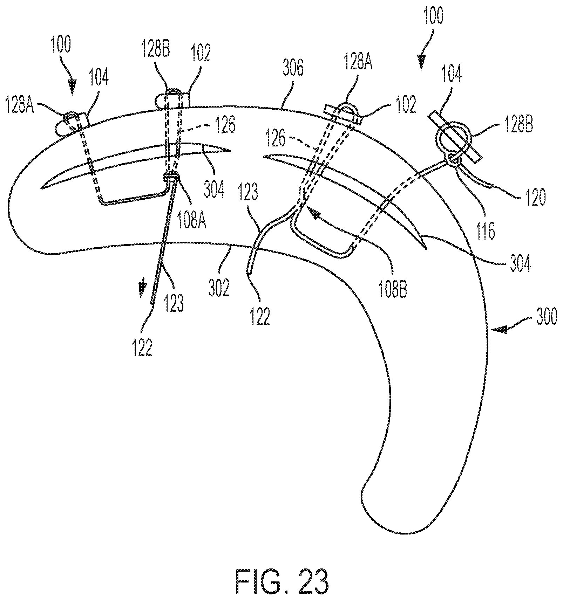

Referring now to FIGs. 23-25, there are shown various views schematic representations of the anchor system 100 in various stages between the pre-deployment configuration and deployed configuration, according to an alternative embodiment. As stated above, the anchor system 100 (FIG. 6) is disposed completely within the needle 212 of the delivery device 200. For example, the first implant 102 is loaded distally in the needle 212 relative to the second implant 104 with the pierce hitch tail 121 and the tensioning limb 123 extending proximally therefrom within the delivery device 200. (Note, it is contemplated that multiple anchor systems 100 can be loaded into the delivery device 200 with the first implant 102 of an additional anchor system 100 behind the second implant 104 of the primary anchor system 100).

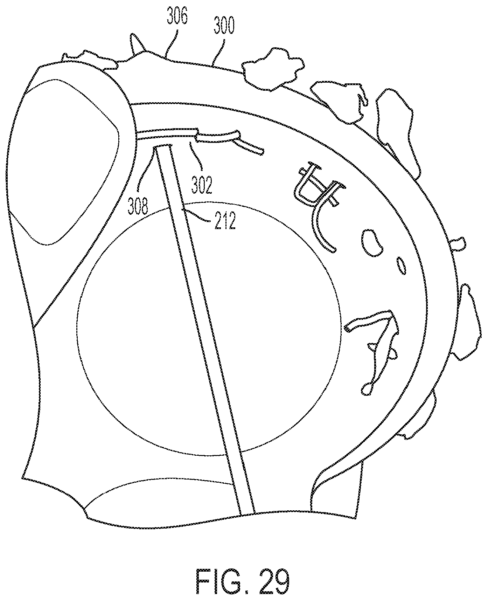

In an embodiment, the pierce hitch tail 121 is releasably connected to the delivery device 200 and functions as a tether used to control the delivery of the second implant 104 and apply traction to the first implant 102. To deploy the anchor system 100, the delivery device 200 is placed on a first side 302 of a tissue 300 or other object. As shown in FIG. 23, the tissue 300 comprises a tear 304 (or cut or other tissue injury). The delivery device 200, in the first configuration, is placed on the first side 302 of the tissue 300 and the tear 304. The needle 212 is advanced through the tissue 300 and the tear 304, at a first piercing location 308, and out of a second, opposing side 306 of the tissue 300 (FIG. 29).

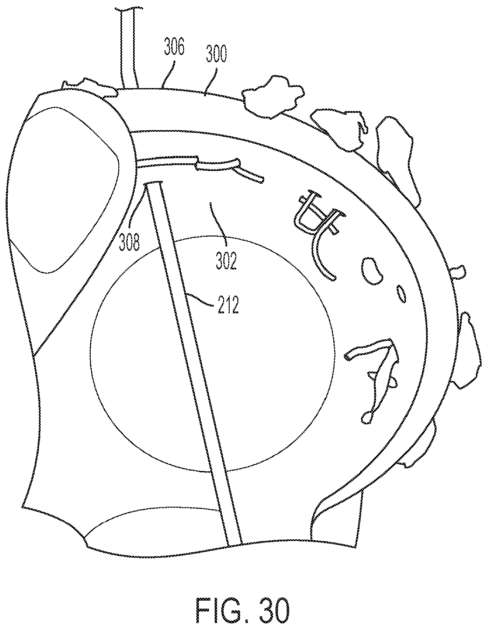

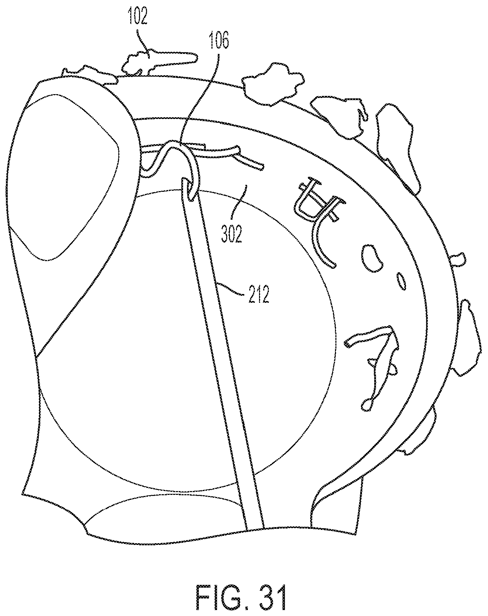

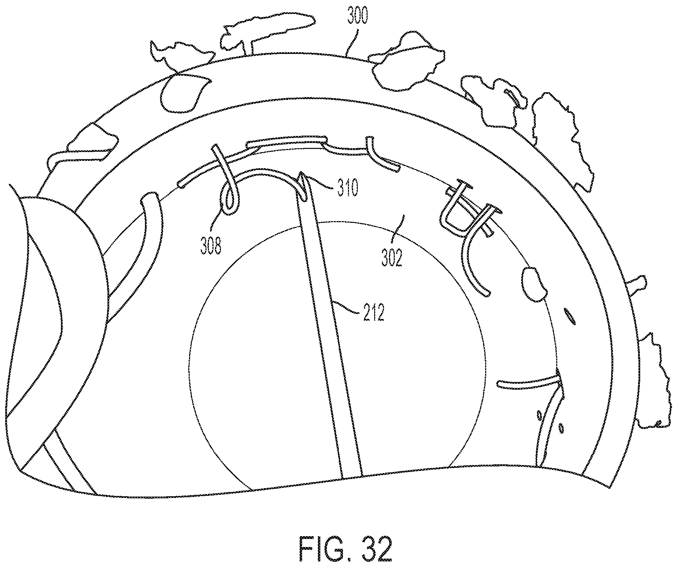

With the needle 212 on the second side 306 of the tissue 300, the actuator 214 is engaged, e.g., the thumb slide 214 is advanced in the distal direction along the elongated body 202, driving the cannulated pusher rod 208 distally to achieve the second configuration. In the second configuration, the cannulated pusher rod 208 pushes the first implant 102 from the delivery device 200 and it is deployed on the second side 306 of the tissue 300, as shown in FIG. 23. The needle 212 is then withdrawn from the first piercing location 308 on the first side 302 of the tissue 300 (FIGs. 30 and 31). In the embodiment of the delivery device 200 shown in FIGs. 18-22, the suture 106 can be tractioned by the tensioning wheel 221 as the needle 212 is withdrawn. The needle 212 is then moved to a second piercing location 310 (adjacent to the first piercing location 308) on the first side 302 of the tissue 300 and tear 304 (FIG. 32).

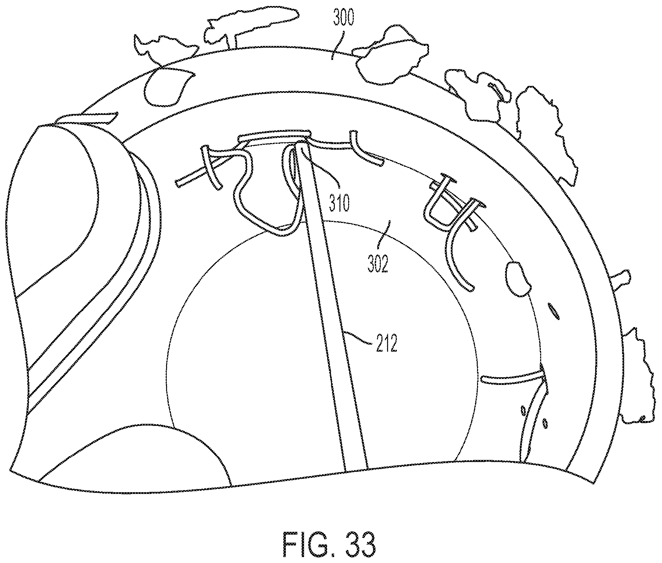

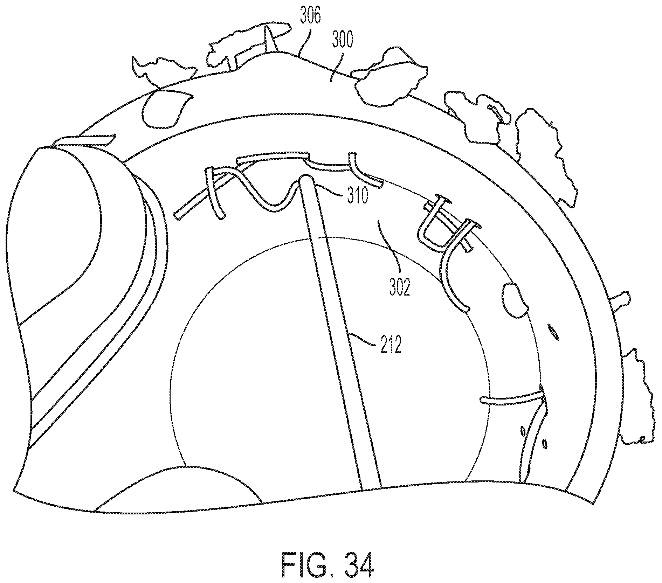

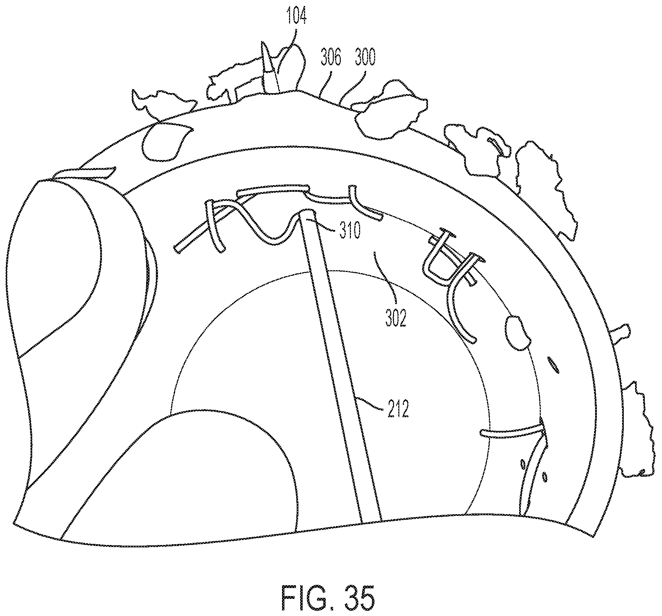

The needle 212 is then advanced through the first side 302, the tear 304, and the second side 306 of the tissue 300 at the second piercing location 310 (FIGs. 33-35). With the needle 212 on the second side 306 of the tissue 300, the delivery device 200 is moved to the third and fourth configurations. From the second configuration, the lockout switch 216 is depressed or otherwise engaged to allow for additional distal movement of the actuator 114. With the lockout switch 216 in the unlocked position, the actuator 114 is moved distally to the third configuration, driving the cannulated pusher rod 208 distally again. In the third configuration, the cannulated pusher rod 208 pushes the second implant 104 from the delivery device 200 and it is deployed, as also shown in FIG. 23.

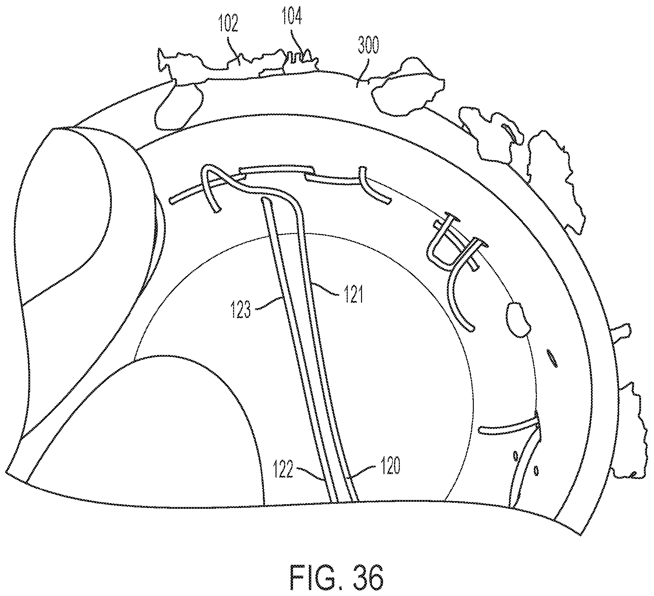

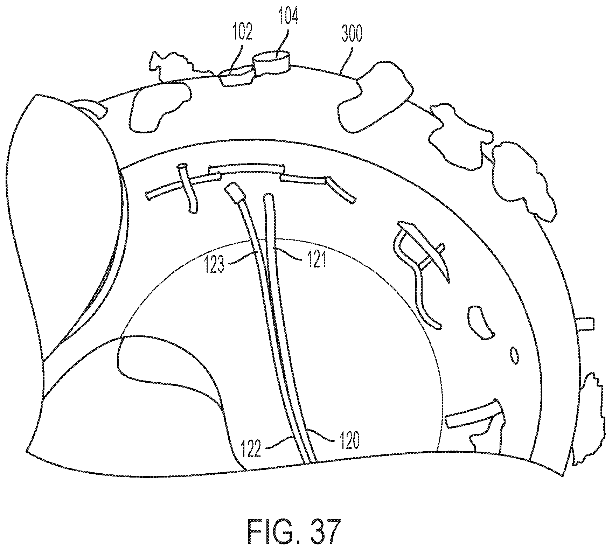

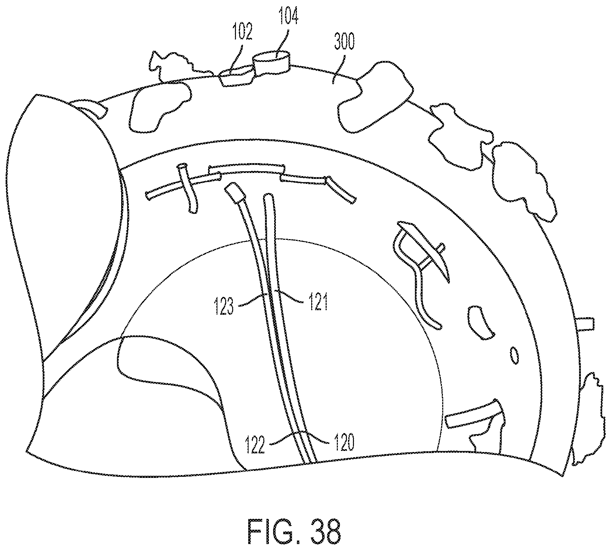

Any time after the second implant 104 is deployed, the pierce hitch tail 121 can be released (i.e., releasing the tether and traction), allowing for the delivery device 200 to be removed, i.e., the needle 212 is pulled back through the second piercing location 310 to the first side 302 of the tissue 300. With the anchor system 100 deployed, the anchor system 100 can be used to move the first side 302 of the tissue 300 and the second side 306 of the tissue 300 together in order to close the tear 304. To do this, the adjustable loop 126 is tightened (i.e., a diameter of the adjustable loop 126 decreases) or is otherwise collapsed by pulling/tensioning the second end 122 (or the tensioning limb 123) of the suture 106 (FIGs. 36-38). Tensioning the second end 122 pulls both implants 102, 104 toward the second side 306 of the tissue 300, decreasing the length of suture 106 between them. In FIG. 23, there are shown two anchor systems 100, each with a different adjustment mechanism 108. The anchor system 100 on the left comprises a sliding knot 108A and the anchor system 100 on the right comprises an eye splice 108B. The eye splice 108B (and the sliding knot 108A) locks the first implant 102 in relative position to the second implant 104. The pierce hitch 116 maintains the position of the second implant 104 along the suture 106.

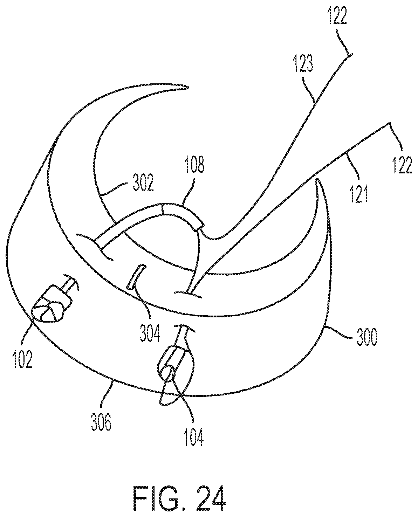

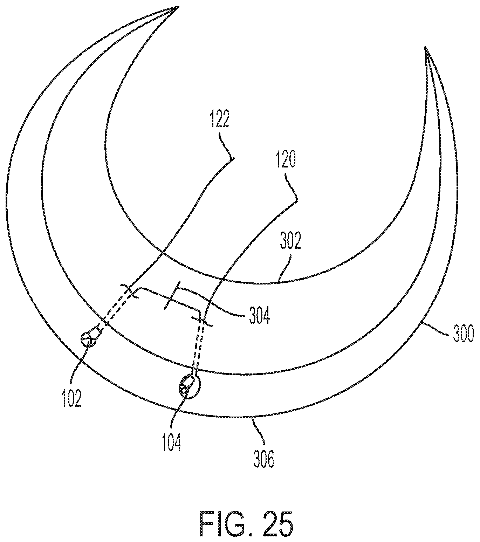

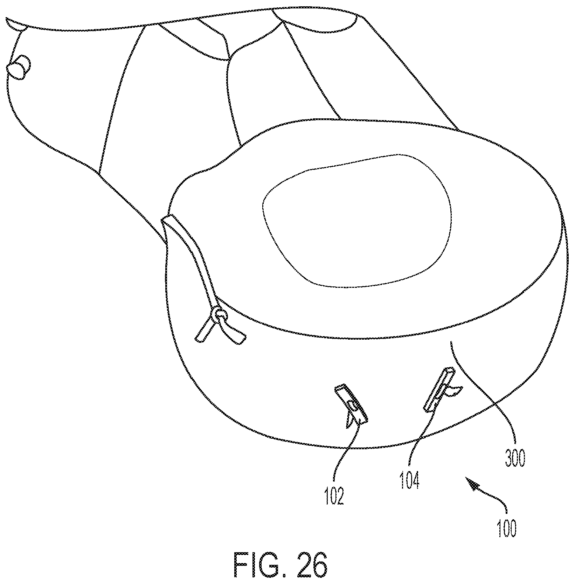

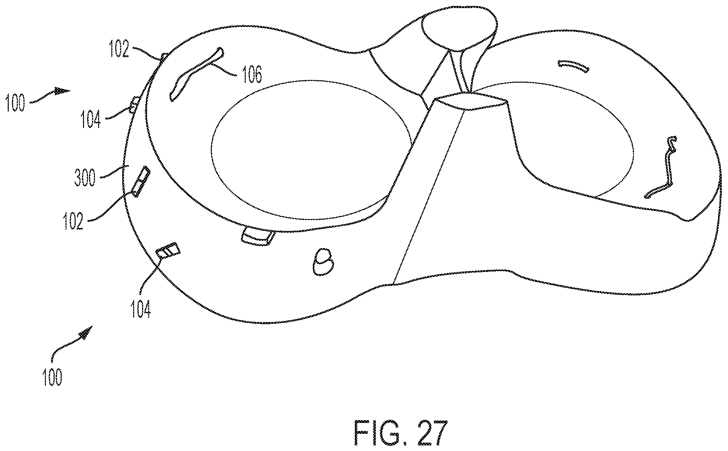

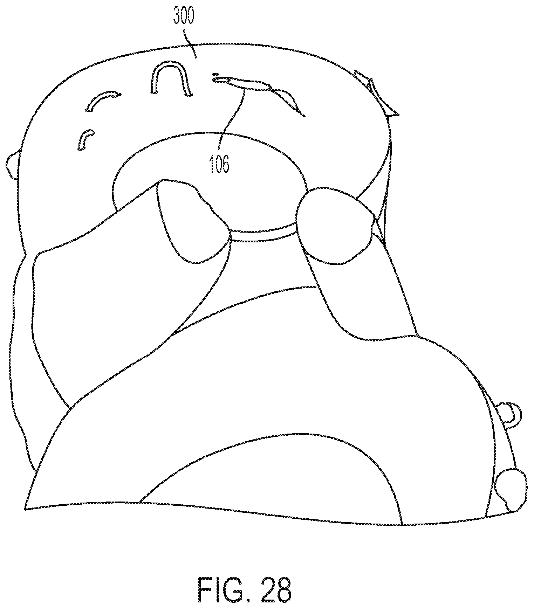

After the desired compression is achieved (between the first implant 102 and the second implant 104) and with the delivery device 200 still in the third configuration, the relieved area 122 is exposed between cannulated pusher rod 208 and the needle 212. The delivery device 200 can be rotated or otherwise maneuvered to receive the excess tensioning limb 123 in the relived area 122. The actuator 114 can then be engaged again, e.g., by sliding the thumb slide 114 back in the proximal direction (toward the proximal end 204 of the elongated body 202). Moving the actuator 214 proximally, pulls the cannulated pusher rod 208 proximally into the needle 212, allowing the excess tensioning limb 123 to be cut against the needle 212 and removed. The same process can be repeated (or occur simultaneously) with any excess pierce hitch tail 121 remaining. The resulting deployed configuration of the anchor system 100 is shown in FIGs. 26-28. FIGs. 24 and 25 show alternative embodiments wherein the implants 102, 104 are cannulated anchors.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, a method or device that "comprises", "has", "includes" or "contains" one or more steps or elements. Likewise, a step of method or an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

The corresponding structures, materials, acts and equivalents of all means or step plus function elements in the claims below, if any, are intended to include any structure, material or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope of the invention. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated.

## Claims

1. An anchor system, comprising:
a first implant (102) connected to a length of suture (106), the length of suture folded such that a tensioning limb (123) extends from the first implant to a second end (122) and a locking limb (121) extends from the first implant to a first end (120);
a second implant (104); and
an adjustment mechanism (108) in the length of suture between the first implant (102) and the second implant (104),
wherein the tensioning limb (123) is passed through the adjustment mechanism (108), and
the second implant (104) is fixed to the locking limb (121) via a pierce hitch (116) in the locking limb,
**characterized in that**
the pierce hitch (116) is formed by inserting the end (120) of the locking limb (121) through a hole in the locking limb,
both the first and second implants (102, 104) are configured to lie flat against damaged tissue, and
the length of suture (106) is connected to the second implant (104) by its second end (122) passing through one of a pair of apertures (110) in the second implant (104), then through the other of the pair of apertures (110) in the second implant (104) and then through the pierce hitch (116).

2. The system of claim 1, further comprising an adjustment loop (126) in the length of a suture extending from the adjustment mechanism (108) through the first implant (102).

3. The system of claim 1, wherein the adjustment mechanism is an eye splice (108).

4. The system of claim 3, wherein the eye splice (108) is in the locking limb (121).

5. The system of claim 1, wherein the first implant (102) and the second implant (104) are rectangular.

6. The system of claim 1, comprising a pair of spaced, adjacent apertures (110) in each of the first and second implants (102, 104).

7. The system of claim 6, further comprising a radiused saddle (112) between each aperture (110) of the pair of spaced, adjacent apertures.

8. The system of claim 6, wherein the length of suture (106) extends through the pair of spaced, adjacent apertures (110) in each of the first and second implants (102, 104).

## Patentansprüche

1. Ankersystem, umfassend:
ein erstes Implantat (102), verbunden mit einer Fadenlänge (106), wobei die Fadenlänge so gefaltet ist, dass ein Spannschenkel (123) vom ersten Implantat zu einem zweiten Ende (122) verläuft und ein Sperrschenkel (121) vom ersten Implantat zu einem ersten Ende (120) verläuft,
ein zweites Implantat (104) und
einen Einstellmechanismus (108) in der Fadenlänge zwischen dem ersten Implantat (102) und dem zweiten Implantat (104),
wobei der Spannschenkel (123) durch den Einstellmechanismus (108) geführt ist und
das zweite Implantat (104) am Sperrschenkel (121) über einen Durchstichknoten (116) im Sperrschenkel befestigt ist,
**gekennzeichnet dadurch, dass**
der Durchstichknoten (116) durch Einführen des Endes (120) des Sperrschenkels (121) durch ein Loch im Sperrschenkel gebildet ist,
wobei sowohl das erste als auch das zweite Implantat (102, 104) dazu eingerichtet sind, flach an beschädigtem Gewebe anzuliegen, und
die Fadenlänge (106) mit dem zweiten Implantat (104) verbunden ist, indem ihr zweites Ende (122) durch eine Öffnung eines Paares von Öffnungen (110) im zweiten Implantat (104), dann durch die andere des Paares von Öffnungen (110) im zweiten Implantat (104) und dann durch den Durchstichknoten (116) geführt ist.

2. System nach Anspruch 1, weiter umfassend eine Einstellschlaufe (126) in der Fadenlänge, die vom Einstellmechanismus (108) durch das erste Implantat (102) verläuft.

3. System nach Anspruch 1, wobei der Einstellmechanismus ein Augenspleiß (108) ist.

4. System nach Anspruch 3, wobei sich der Augenspleiß (108) im Sperrschenkel (121) befindet.

5. System nach Anspruch 1, wobei das erste Implantat (102) und das zweite Implantat (104) rechteckig sind.

6. System nach Anspruch 1, umfassend ein Paar beabstandeter, benachbarter Öffnungen (110) in jedem der ersten und zweiten Implantate (102, 104).

7. System nach Anspruch 6, ferner umfassend einen gerundeten Sattel (112) zwischen jeder Öffnung (110) des Paares beabstandeter, benachbarter Öffnungen.

8. System nach Anspruch 6, wobei die Fadenlänge (106) durch das Paar beabstandeter, benachbarter Öffnungen (110) in jedem der ersten und zweiten Implantate (102, 104) verläuft.

## Revendications

1. Système d'ancrage, comprenant :
un premier implant (102) connecté à une longueur de suture (106), la longueur de suture pliée de telle façon qu'un brin (123) de mise en tension s'étend depuis le premier implant jusqu'à une deuxième extrémité (122) et un brin (121) de verrouillage s'étend depuis le premier implant jusqu'à une première extrémité (120) ;
un deuxième implant (104) ; et
un mécanisme (108) d'ajustement de la longueur de suture entre le premier implant (102) et le deuxième implant (104),
dans lequel le brin (123) de mise en tension est passé à travers le mécanisme (108) d'ajustement, et
le deuxième implant (104) est fixé au brin (121) de verrouillage par l'intermédiaire d'un nœud ajouré (116) dans le brin de verrouillage,
**caractérisé en ce que**
le nœud ajouré (116) est formé en insérant l'extrémité (120) du brin (121) de verrouillage à travers un trou dans le brin de verrouillage,
les premier et deuxième implants (102, 104) sont tous les deux configurés pour reposer à plat contre le tissu endommagé, et
la longueur de suture (106) est connectée au deuxième implant (104) par sa deuxième extrémité (122) passant à travers une d'une paire d'ouvertures (110) dans le deuxième implant (104), ensuite à travers l'autre de la paire d'ouvertures (110) dans le deuxième implant (104) et ensuite à travers le nœud ajouré (116).

2. Système selon la revendication 1, comprenant en outre une boucle (126) d'ajustement dans la longueur d'une suture s'étendant depuis le mécanisme (108) d'ajustement à travers le premier implant (102).

3. Système selon la revendication 1, dans lequel le mécanisme d'ajustement est une épissure (108) à œillet.

4. Système selon la revendication 3, dans lequel l'épissure (108) à œillet est dans le brin (121) de verrouillage.

5. Système selon la revendication 1, dans lequel le premier implant (102) et le deuxième implant (104) sont rectangulaires.

6. Système selon la revendication 1, comprenant une paire d'ouvertures adjacentes espacées (110) dans chacun des premier et deuxième implants (102, 104).

7. Système selon la revendication 6, comprenant en outre une sellette arrondie (112) entre chaque ouverture (110) de la paire d'ouvertures adjacentes espacées.

8. Système selon la revendication 6, dans lequel la longueur de suture (106) s'étend à travers la paire d'ouvertures adjacentes espacées (110) dans chacun des premier et deuxième implants (102, 104).
